# EUROPÄISCHE PATENTSCHRIFT

(11) **EP 1 648 321 B1**
(45) Veröffentlichungstag und Bekanntmachung des Hinweises auf die Patenterteilung: **31.12.2008**
(21) Anmeldenummer: 03739952.4
(22) Anmeldetag: 30.07.2003
(51) Int. Cl.: A61B 17/72

(54) **CHIRURGISCHER NAGEL**
SURGICAL NAIL
CLOU CHIRURGICAL

(43) Veröffentlichungstag der Anmeldung: 26.04.2006
(73) Patentinhaber: Synthes GmbH, 4436 Oberdorf (CH)
(72) Erfinder: SCHLIENGER, André, CH-4142 Münchentein (CH); HÖNTZSCH, Dankward, 72076 Tübingen (DE); BUETTLER, Markus, CH-4702 Oensingen (CH); SENN, Peter, CH-4437 Waldenburg (CH)
(74) Vertreter: Lusuardi, Werther
(86) Internationale Anmeldenummer: PCT/CH2003/000519
(87) Internationale Veröffentlichungsnummer: WO 2005/009263

(56) Entgegenhaltungen:
- FR-A- 2 784 283
- US-A1- 2002 173 792
- US-A1- 2003 114 855

## Beschreibung

Die Erfindung betrifft einen chirurgischen Nagel, insbesondere einen intramedullären Marknagel, gemäss dem Oberbegriff des Patentanspruchs 1.

Die Verriegelung von Marknägeln gehört zum Stand der Technik. Die Einführung der Verriegelungsschrauben oder Verriegelungsbolzen (im folgenden wird nur noch der Begriff Verriegelungsschraube verwendet, der aber auch den Begriff Verriegelungsbolzen mitumfassen soll) in die Querbohrungen des Marknagels erfolgt entweder mit Hilfe eines bildgebenden Verfahrens (Röntgenkontrolle) oder einer mehr oder weniger komplizierten Zielvorrichtung. In beiden Fällen ist eine gewisse Zielungenauigkeit nicht zu vermeiden, d.h. die Schraubenspitze lässt sich nicht exakt koaxial zur Mittelachse der Querbohrung ausrichten, sondern weicht davon um einen gewissen Betrag ab. Damit die Verriegelungsschraube trotz dieses Zielfehlers in die Querbohrung mündet und durch diese hindurchgebracht werden kann, wird der Aussendurchmesser der Schraube relativ zum Durchmesser der Querbohrung unterdimensioniert. Bleibt die Zielungenauigkeit im Rahmen dieser Unterdimensionierung, so kann die Verriegelungsschraube trotz des Zielfehlers problemlos durch die Querbohrung geführt werden. Allerdings weist nun die Verriegelungsschraube - wegen der Unterdimensionierung - relativ zur Querbohrung ein gewisses Spiel auf.

Dieses Spiel definiert, um welchen Betrag sich die Knochenhauptfragmente, welche mittels Verriegelungsschrauben im entsprechenden Verriegelungsloch fixiert werden, relativ zum Nagel und somit aufgrund der Starrheit des Nagels auch relativ zu anderen mit demselben Nagel befestigten Knochenhauptfragmenten bewegen können. Um die Anwendbarkeit der Verriegelung für den Chirurgen zu garantieren, ist dieses Spiel zwar unumgänglich, doch ist es klinisch bei gewissen Indikationen (z.B. im Falle von metaphysären Fragmenten) unerwünscht.

Selbst Nägel mit vollem Querschnitt, welche im Verriegelungsloch ein Innengewinde aufweisen können, sind nicht spielfrei. Das Innengewinde verhindert lediglich, dass der Nagel sich axial auf der Verriegelungsschraube verschieben kann.

Aus der US 6,296,645 HOVER ET AL. ist ein hohler, intramedullärer Marknagel aus Metall bekannt, der in den als Fenster bezeichneten sich diametral gegenüberstehenden Mantelöffnungen der Querbohrung einen oder zwei Kunststoffeinsätze aufweist, durch welche die Verriegelungsschraube eingeführt werden kann. Nachteilig bei diesem bekannten Marknagel ist der Umstand, dass die fensterartigen Kunststoffeinsätze leicht eingedrückt werden können, so dass die erwünschte Funktion verloren geht. Aber selbst bei einer sehr vorsichtigen Manipulation dürften die beiden Kunststoffeinsätze beim Durchführen der Verriegelungsschraube aus ihrem "Fenster" gedrückt werden, was ebenfalls zu einem Verlust der Funktion führt.
Ein weiterer Marknagel mit einer Querbohrung und einem in dieser angeordneten Einsatz, durch welchen eine Verriegelungsschraube durchführbar ist, ist aus der US-A 2002/0173792 SEVERNS bekannt. Der Oberbegriff vom Anspruch 1 basiert auf ihrem Inhalt.
Ein anderer Marknagel mit einer Querbohrung und einem in dieser angeordneten Einsatz, durch welchen eine Verriegelungsschraube durchführbar ist, ist aus der FR 2 784 283 AARON bekannt.
Hier will die Erfindung Abhilfe schaffen. Der Erfindung liegt die Aufgabe zugrunde, einen chirurgischen Nagel, insbesondere einen intramedullären Marknagel zu schaffen, mit dem das vorhandene Spiel zwischen ihm und der Verriegelungsschraube risikolos eliminiert werden kann und eine verbesserte Haltekraft sowie ein verbesserter Führungseffekt zwischen Verriegelungsschraube und Marknagel erzielt werden kann.

Die Erfindung löst die gestellte Aufgabe mit einem chirurgischen Nagel, welcher die Merkmale des Anspruchs 1 aufweist.
Damit sind folgende Vorteile erzielbar:
a) die Zielgenauigkeit bei der Einbringung der Verriegelungsschraube ist unbeeinträchtigt;
b) es besteht die Möglichkeit der winkelstabilen Fixierung des Knochenfragments in gewissen Richtungen für einen bestimmten Betrag der Last;
c) das Bauelement kann nach dem Aufbohren der Kortikalis in den Marknagel eingebracht werden, d.h. es entstehen keine Fremdspäne im Markkanal; und
d) je nach Auswahl des Bauelementes kann eine stärkere oder schwächere Winkelstabilität der Verriegelungsschraube erreicht werden.

Bei einer besonderen Ausführungsform besteht das Bauelement aus einem Stift, einem Drahtstück oder einem Kabelstück, welches im wesentlichen parallel zur Längsachse des Nagels ausgerichtet ist.

Das Bauelement besteht bei einer bevorzugten Ausführungsform aus dem gleichen Material wie der Marknagel, d.h. aus Titan oder einer Titanlegierung für einen Titan-Nagel oder aus Stahl für einen Stahlnagel.

Das Bauelement kann aber auch aus einem biokompatiblen Kunststoff, z.B. einem Polyethylen, insbesondere aus einem hochmolekularen Polyethylen (HMWPE) bestehen. Der Vorteil dieser Materialien besteht darin, dass sie einerseits nicht allzu hart sind und anderseits kein Abbau des Kunststoffs mit unbekannten Abbauprodukten resultiert.
Der Kunststoff kann aber auch ein bioresorbierbares Polymer, vorzugsweise ein Polylactid sein. Bei dieser Ausführung resultiert anfänglich eine spielfreie Querverriegelung des Marknagels, welche dann mit zunehmender Resorption des Polymers sukzessive wieder aufgehoben wird, so dass die Querverriegelungsschraube relativ zum Marknagel und somit auch die versorgten Knochenfragmente wieder beweglich werden. Es erfolgt somit eine Dynamisierung der Knochenfragmente nach erfolgter Frakturkonsolidierung.
Ein weiterer Vorteil des bioresorbierbaren Material ist der Umstand, dass die beim Hindurchschrauben einer Verriegelungsschraube durch die Querbohrung des Nagels eventuell entstehenden Späne vom Körper abgebaut werden können.

Die Querbohrung des Nagels kann entweder als Kreisbohrung (a = b) oder als Langloch (a > b) ausgebildet sein.

Die in die Querbohrung einführbare Verriegelungsschraube (oder ein Verriegelungsbolzen) weist zweckmässigerweise einen Schaft auf mit einem Durchmesser d, wobei a > d < b. Im Falle einer Verriegelungsschraube umfasst der Durchmesser d auch deren Aussengewinde. Der Durchmesser d ist zudem vorzugsweise mindestens 5 % kleiner ist als die kleinere der beiden Dimensionen a, b des Querschnittsprofils F.

Das Bauelement lässt sich an verschiedenen Orten innerhalb des Marknagels anbringen, bzw. befestigen. Vorzugsweise wird es jedoch an der Innenfläche der Wandung des Marknagels befestigt.

Die Erfindung und Weiterbildungen der Erfindung werden im folgenden anhand der teilweise schematischen Darstellung mehrerer Ausführungsbeispiele noch näher erläutert.

Es zeigen:
Fig. 1 eine perspektivische Ansicht des erfindungsgemässen Marknagels;
Fig. 2 einen Schnitt durch den Marknagel längs der Linie II-II in Fig. 1;
Fig. 3 einen Längsschnitt durch eine Variante des Marknagels;
Fig. 4 einen Längsschnitt durch eine weitere Variante des Marknagels; und
Fig. 5 einen Schnitt durch den Markangel längs der Linie V-V in Fig. 4.

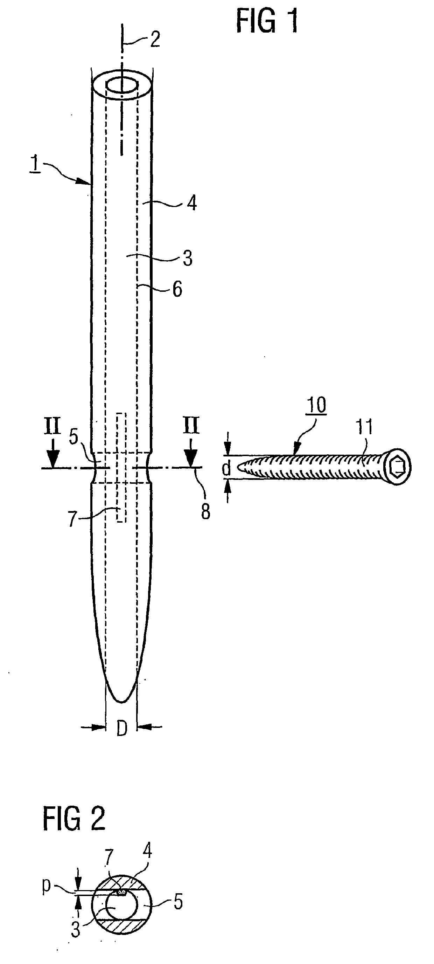

Der in den Fig. 1 und 2 dargestellte chirurgische Nagel 1 ist ein intramedullärer Marknagel für Röhrenknochen mit einer Zentralachse 2 der aus einem Metall oder einer Metalllegierung, d.h. einem Material mit relativ hoher Festigkeit besteht und einer quer zur Zentralachse 2 verlaufenden Querbohrung 5 mit dem Querschnittsprofil F und der Querachse 6. Die Querbohrungen 5 ist proximal angebracht und als Kreisbohrung ausgebildet. Sie könnte aber auch als Langloch mit den maximalen Dimensionen a und b ausgebildet sein wobei die längere Dimension a in axialer Richtung angeordnet wäre.

Der Marknagel besitzt eine koaxial zur Zentralachse 2 verlaufende Längsbohrung 3 und dadurch einen Mantel 4 mit der Innenfläche 6. In der Längsbohrung 3 ist im Bereich der Querbohrung 5 ein das Querschnittsprofil F der Querbohrung 5 verengendes Bauelement 7 an der Innenfläche 6 des Mantels 4 befestigt. Im dargestellten Beispiel ist das Bauelement 7 im hinteren Bereich des Längsbohrung 3 angebracht und weist eine vorspringende Dicke P auf. Die Dicke P ist dabei derart gewählt, dass die Verriegelungsschraube 10 mit dem Aussengewinde 11 gerade noch in die Querbohrung 5 eingedreht werden kann.

Das Bauelement 7 besteht aus einem Stift, der im wesentlichen parallel zur Längsachse 2 ausgerichtet ist. Statt eines Stiftes kann auch ein anderes longitudinales Bauelement verwendet werden, wie z.B. ein Drahtstück, Kabelstück oder eine Profilstück.

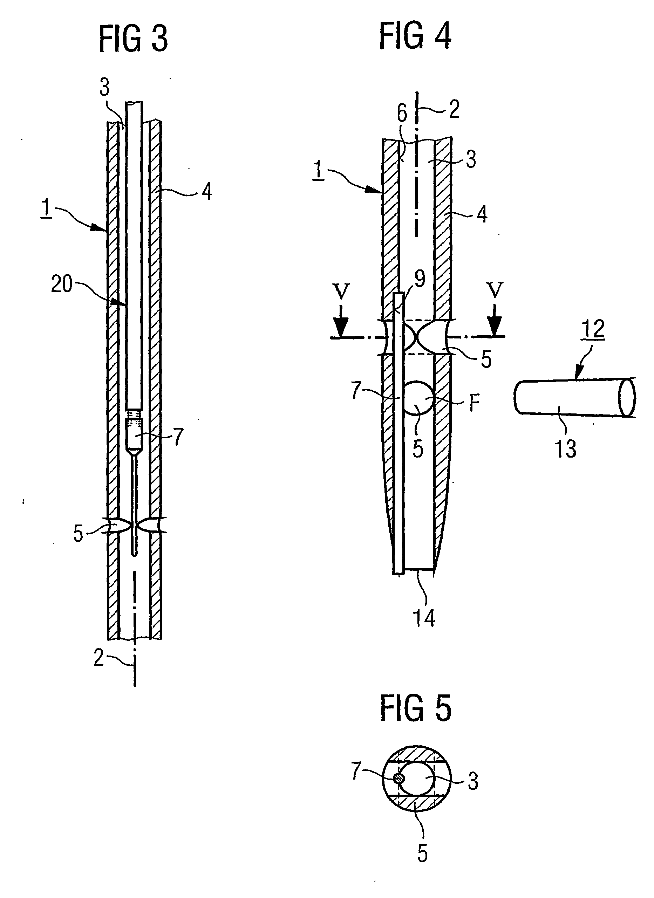

In Fig. 3 ist eine Variante eines erfindungsgemässen Marknagels dargestellt, bei welcher das Bauelement 7 als separates, mittels eines geeigneten Instrumentes 20 intraoperativ in die Längsbohrung 3 des Marknagels 1 einführbares Element ausgebildet ist.

In den Fig. 4 und 5 ist eine weitere Variante eines erfindungsgemässen Marknagels dargestellt, bei welcher die Innenfläche 6 des Mantels 4 eine parallel zur Zentralachse 2 verlaufende Nut 9 aufweist, in welche das Bauelement 7 von der Spitze 14 des Marknagels 1 her in die Längsbohrung 3 einführbar ist. Die Länge des Bauelementes 7 ist derart bemessen, dass es bei vollständiger Einführung in den Marknagel 1 in den Bereich der Querbohrung 5 gelangt, so dass deren Querschnittsprofil F durch das Bauelement 7 verengt wird. Die Nut 9 weist einen teilkreisförmigen Querschnitt auf mit einem Zentriwinkel von 200°. Der Durchmesser des Bauelementes 7 ist dabei derart dimensioniert, dass sein radial in die Längsbohrung 6 vorspringender Anteil eine solche Verengung des Querschnittsprofils F bewirkt, dass der Durchgang des Verriegelungsbolzens 12 mit dem glatten Schaft 13 gerade noch möglich ist.

## Patentansprüche

1. Chirurgischer Nagel (1), insbesondere intramedullärer Marknagel, mit einer Zentralachse (2), einer koaxial zur Zentralachse (2) verlaufenden Längsbohrung (3) mit dem Durchmesser D, einem Mantel (4) mit der Innenfläche (6) und einer quer zur Zentralachse (2) verlaufenden Querbohrung (5), welche geeignet ist eine Verriegelungsschraube (10) aufzunehmen, wobei die Querbohrung ein Querschnittsprofil F und eine Bohrungsachse (8) aufweist; wobei
A) die Längsbohrung (3) durchgehend ist;
B) in der Längsbohrung (3) im Bereich der Querbohrung (5) ein das Querschnittsprofil F verengendes Bauelement (7) vorgesehen ist, welches den Durchgang einer Verriegelungsschraube (10) noch zulässt;
C) das Bauelement (7) einen radial in die Längsbohrung (6) vorspringenden Anteil der Dicke P > 0,01 D aufweist;
D) das Bauelement ein longitudinales Bauelement ist, welches im wesentlichen parallel zur Längsachse ausgerichtet ist; und
**dadurch gekennzeichnet, dass**
E) das Bauelement (7) aus dem gleichen metallischen Material besteht wie der Nagel (1).

2. Nagel (1) nach Anspruch 1, **dadurch gekennzeichnet, dass** das Bauelement (7) an der Innenfläche (6) des Mantels (4) befestigt ist.

3. Nagel (1) nach Anspruch 1 oder 2, **dadurch gekennzeichnet, dass** das Bauelement (7) ein Stift, Drahtstück, Kabelstück oder Profilstück ist und im wesentlichen parallel zur Längsachse (2) ausgerichtet ist.

4. Nagel (1) nach einem der Ansprüche 1 bis 3, **dadurch gekennzeichnet, dass** das Querschnittsprofil F in Richtung der Zentralachse (2) eine maximale Länge a und senkrecht dazu eine maximale Breite b aufweist, wobei a > b gilt.

5. Nagel (1) nach einem der Ansprüche 1 bis 4, **dadurch gekennzeichnet, dass** die Querbohrung (5) eine Kreisbohrung ist, wobei das Querschnittsprofil F die maximalen Längen a =b aufweist.

6. Nagel (1) nach einem der Ansprüche 1 bis 5, **dadurch gekennzeichnet, dass** er eine in die Querbohrung (5) einführbare Verriegelungsschraube (10) mit einem Aussengewinde (11) oder einen Verriegelungsbolzen (12) mit einem glatten Schaft (13) umfasst, wobei das Aussengewinde (11), bzw. der Schaft (13) einen Durchmesser "d" aufweisen und die Bedingung a > d < b gilt.

7. Nagel (1) nach Anspruch 6, **dadurch gekennzeichnet, dass** der Durchmessers "d" vorzugsweise mindestens 5 % kleiner ist als die kleinere der beiden Dimensionen a, b.

8. Nagel (1) nach einem der Ansprüche 1 bis 7, **dadurch gekennzeichnet, dass** er zwei sich durchdringende Querbohrung (5) aufweist und das Bauelement (7) vorzugsweise im Eckbereich der Querbohrungen (5) positioniert ist.

9. Nagel (1) nach einem der Ansprüche 1 bis 8, **dadurch gekennzeichnet, dass** das Bauelement (7) als separates, intraoperativ in die Längsbohrung (3) einführbares Element ausgebildet ist.

10. Nagel (1) nach Anspruch 9, **dadurch gekennzeichnet, dass** die Innenfläche (6) des Mantels (4) eine parallel zur Zentralachse (2) verlaufende Nut (9) aufweist, in welche das Bauelement (7) einführbar ist.

11. Nagel nach Anspruch 10, **dadurch gekennzeichnet, dass** die Nut (9) einen teilkreisförmigen Querschnitt aufweist, vorzugsweise mit einem Zentriwinkel der grösser als 180° ist.

12. Nagel nach einem der Ansprüche 1 bis 11, **dadurch gekennzeichnet, dass** das Bauelement (7) eine radial in die Längsbohrung vorspringende Dicke P > 0,01 D, vorzugsweise P > 0,05 D aufweist.

## Claims

1. Surgical pin (1), especially an intramedullary pin, with a central axis (2), a longitudinal borehole (3) extending coaxially with the central axis (2) with the diameter D, a casing (4) with the inner surface (6) and a transverse borehole (5) extending transversely to the central axis (2), which is suitable for accommodating a locking screw (10), the transverse borehole having a cross-sectional profile F and a borehole axis (8),
A) the longitudinal borehole (3) is continuous;
B) a component (7), narrowing the cross-sectional profile F but still permitting passage of a locking screw (10), is provided in the longitudinal borehole (3) in the region of the transverse borehole (5);
C) the component (7) comprises a part which radially protrudes into the longitudinal borehole (3) and has a thickness P > 0,01 D; and
D) the component (7) is a longitudinal component, which is aligned essentially parallel to the longitudinal axis (2),
**characterized in that**
E) the component (7) consists of the same metallic material as the pin (1).

2. The pin (1) of claim 1, **characterized in that** the component (7) is fastened to the inner surface (6) of the casing (4).

3. The pin (1) of claims 1 or 2, **characterized in that** the component (7) is a stud, a piece of wire, a piece of cable or a profiled piece and is aligned essentially parallel to the longitudinal axis (2).

4. The pin (1) of one of the claims 1 to 3, **characterized in that** the cross-sectional profile F has a maximum length a in the direction of the central axis (2) and, perpendicularly thereto, a maximum width b with a > b.

5. The pin (1) of one of the claims 1 to 4, characterizing that the transverse borehole (5) is a circular borehole, the cross-sectional profile F having the maximum lengths a = b.

6. The pin (1) of one of the claims 1 to 5, **characterized in that** it comprises a locking screw (10) with an external thread (11), which can be introduced into the transverse borehole (5), or a locking bolt (12) with a smooth shaft (13), the external thread (11) or the shaft (13) having a diameter "d" satisfying the condition a >d<b.

7. The pin (1) of claim 6, **characterized in that** the diameter "d" preferably is at least 5% smaller than the smaller of the two dimensions a, b.

8. The pin (1) of one of the claims 1 to 7, **characterized in that** it has two penetrating, transverse boreholes (5) and the component (7) is positioned preferably in the corner region of the transverse boreholes (5).

9. The pin (1) of one of the claims 1 to 8, **characterized in that** the component (7) is constructed as a separate element, which can be introduced into the longitudinal borehole (3) during the operation.

10. The pin (1) of claim 9, **characterized in that** the inner surface (6) of the casing (4) has a groove (9), which extends parallel to the central axis (2) and into which the component (7) can be introduced.

11. The pin (1) of claim 10, **characterized in that** the groove (9) has a partially circular cross section, preferably with a central angle, which is larger than 180°.

12. The pin (1) of one of the claims 1 to 11, **characterized in that** the component (7) has a thickness of P > 0.01 D and preferably of P > 0.05 D protruding radially into the longitudinal borehole.

## Revendications

1. Clou chirurgical (1), notamment clou intramédullaire, lequel comporte un axe central (2), un perçage longitudinal (3) de diamètre D qui s'étend coaxialement à l'axe central (2), une enveloppe (4) ayant une surface intérieure (6) et un perçage transversal (5) qui s'étend transversalement à l'axe central (2) et qui est approprié à recevoir une vis de verrouillage (10), le perçage transversal ayant un profil F en coupe transversale et un axe de perçage (8) ;
A) le perçage longitudinal (3) étant ménagé de bout en bout,
B) un élément structurel (7), qui réduit le profil F en coupe transversale et qui permet encore le passage d'une vis de verrouillage (10), étant prévu dans le perçage longitudinal (3) dans la région du perçage transversal (5) ;
C) l'élément structurel (7) comportant une partie qui fait saillie dans le perçage longitudinal, d'épaisseur P > 0,01 D ;
D) l'élément structurel étant un élément structurel longitudinal qui est orienté sensiblement parallèlement à l'axe longitudinal ; et
**caractérisé en ce que**
E) l'élément structurel (7) est en le même matériau métallique que le clou (1).

2. Clou (1) selon la revendication 1, **caractérisé en ce que** l'élément structurel (7) est fixé à la surface intérieure (6) de l'enveloppe (4).

3. Clou (4) selon la revendication 1 ou 2, **caractérisé en ce que** l'élément structurel (7) est une cheville, un morceau de fil, un morceau de câble ou une pièce profilée et est orienté sensiblement parallèlement à l'axe longitudinal (2).

4. Clou (1) selon l'une des revendications 1 à 3, **caractérisé en ce que** le profil F en coupe transversale précédente, dans la direction de l'axe central (2), une longueur maximale a et, perpendiculairement à cette direction, une largeur maximale b, avec a > b.

5. Clou (1) selon l'une des revendications 1 à 4, **caractérisé en ce que** le perçage transversal (5) est un perçage circulaire, le profil F en coupe transversale présentant la longueur maximale a = b.

6. Clou (1) selon l'une des revendications 1 à 5, **caractérisé en ce qu'**il possède une vis de verrouillage (10), qui peut être insérée dans le perçage transversal (5) et qui présente un filetage extérieur (11), ou un boulon de verrouillage (12) dont la tige (13) est lisse, le filetage extérieur (11), respectivement la tige (13), ayant un diamètre 'd' et remplissant la condition a>d<b.

7. Clou (1) selon la revendication 6, **caractérisé en ce que** le diamètre 'd'est inférieur, avantageusement d'au moins 5%, à la plus petite des deux dimensions a, b.

8. Clou (1) selon l'une des revendications 1 à 7, **caractérisé en ce qu'**il comporte deux perçages transversaux (5) qui pénètrent l'un dans l'autre et l'élément structurel (7) est avantageusement positionné au niveau de l'angle des perçages transversaux (5).

9. Clou (1) selon l'une des revendications 1 à 8, **caractérisé en ce que** l'élément structurel (7) est conformé en élément séparé qui peut être inséré dans le perçage longitudinal (3) pendant une opération.

10. Clou (1) selon la revendication 9, **caractérisé en ce que** la surface intérieure (6) de l'enveloppe (4) comporte une gorge (9) qui s'étend parallèlement à l'axe central (2) et dans laquelle l'élément structurel peut être inséré.

11. Clou selon la revendication 10, **caractérisé en ce que** la gorge (9) a une section partiellement circulaire avec un angle au centre qui est supérieur à 180°.

12. Clou selon l'une des revendications 1 à 11, **caractérisé en ce que** l'élément structurel (7) présente une épaisseur P > 0,01 D, avantageusement P > 0,05 D, qui fait saillie dans le perçage longitudinal.
